# EUROPÄISCHE PATENTSCHRIFT

(11) **EP 4 255 996 B1**
(45) Veröffentlichungstag und Bekanntmachung des Hinweises auf die Patenterteilung: **20.11.2024**
(21) Anmeldenummer: 21824521.5
(22) Anmeldetag: 01.12.2021
(51) Int. Cl.: C09G 3/00, C09D 5/00

(54) **MITTEL ZUR VERMINDERUNG DER GLEITREIBUNG EINES GEGENSTANDES AUF SCHNEE, EIS UND/ODER WASSER**
COMPOSITION FOR REDUCING SLIDING FRICTION OF AN ARTICLE ON SNOW, ICE AND/OR WATER
COMPOSITION POUR RÉDUIRE LE FROTTEMENT PAR GLISSEMENT D'UN ARTICLE SUR LA NEIGE, LA GLACE ET/OU L'EAU

(30) Priorität: 01.12.2020 CH 15292020
(43) Veröffentlichungstag der Anmeldung: 11.10.2023
(73) Patentinhaber: ISANTIN GMBH, 9450 Altstätten (CH)
(72) Erfinder: BÜTZER, Marcel, 9450 Altstätten SG (CH); BÜTZER, Peter, 9450 Altstätten SG (CH)
(74) Vertreter: Bogensberger, Burkhard
(86) Internationale Anmeldenummer: PCT/EP2021/083745
(87) Internationale Veröffentlichungsnummer: WO 2022/117637

(56) Entgegenhaltungen:
- EP-A1- 0 696 620
- EP-A1- 2 161 307
- WO-A1-2008/043481
- WO-A1-2015/106095
- WO-A1-2019/145282
- US-A- 5 914 298
- US-A1- 2010 087 345

## Beschreibung

### TECHNISCHES GEBIET

Die vorliegende Erfindung betrifft ein Mittel zur Reduktion von Gleitreibung auf Schnee, Eis und/oder Wasser, welches Chinacridon und/oder substituierte Chinacridone als Gleitmittelkomponente enthält und auf verschiedene Gegenstände und Geräte, insbesondere Wintersportgeräte wie z.B. Skis, Schlitten, Snowboards, oder Wassersportgeräte wie z.B. Boote, Surfbretter, oder Wasserskis, anwendbar ist.

### STAND DER TECHNIK

Die Gleitflächen von Wintersportgeräten wie Alpinskis, Langlaufskis, Sprungskis, Snowboards, Schlitten, Skibobs und dergleichen werden sowohl im Freizeitsport als auch im Leistungssport häufig mit speziellen Gleitmitteln beschichtet, um die Reibung zwischen der Gleitfläche des Sportgerätes und der Kontaktfläche des Untergrunds, nämlich Schnee, Eis, Wasser oder einer Mischung zweier oder aller drei dieser Komponenten, zu verringern und damit vor allem die mit dem Sportgerät erzielbare Geschwindigkeit zu verbessern. Wobei die Mischung eine Mischung aus Schnee/Eis, Schnee/Wasser, Eis/Wasser oder Schnee/Eis/Wasser sein kann.

Entscheidend für gute Gleiteigenschaften einer Gleitfläche ist der chemische und physikalische Aufbau und die Struktur, die mit dem Eis, Schnee, Wasser oder einem Gemisch davon, in Berührung kommt. Im Idealfall entsteht durch den Kontakt von Gleit- und Kontaktfläche nur eine geringe Gleitreibung, selbst bei unterschiedlichen oder sich verändernden Zuständen der Eis-, Schnee- und/oder Wasseroberflächen, auch bei wechselnden äusseren Bedingungen wie Temperatur- und Wetteränderungen, und das vorzugsweise über eine - je nach Sportart - ausreichend lange Zeitdauer der Anwendung. Die physikalisch-chemischen Voraussetzungen an eine Gleitfläche, um ein derartiges Ziel zu erreichen, umfassen einerseits eine möglichst geringe Benetzbarkeit mit Wasser, wenig Abrieb, niedrige Oxidations- sowie Lichtempfindlichkeit, eine minimale Schmutzaufnahme, und andererseits - bei aufgetragener Schicht eines Gleitmittels - eine durchgehende, auf der Gleitoberfläche gut haftende Schicht des Gleitmittels.

Um diesem Ziel möglichst nahe zu kommen, wurden für den Wintersport sehr viele in der Tribologie bekannte Schmiermittel als Gleitmittel oder Gleitmittelbestandteile ausprobiert und weiterentwickelt. Dazu zählen vor allem Teere, Öle, Fette, Fettalkohole und Fettether, Wachse, natürliche, synthetische, mikrokristalline, nichthalogenierte und halogenierte Paraffine, Silikone, silanisierte Partikel, Graphit/Graphen/Nanotubes, Molybdändisulfid, Wolframdisulfid, Wolframdiselenid, Gallium, hexagonales Bornitrid, Polymere, und PTFE.

Auf ökologische und arbeitshygienische Kriterien wurde dabei bedauerlicherweise kaum geachtet. Deshalb werden bis heute die bereits 1986 eingeführten teil- und perfluorierten Paraffine (PFAS, PFC) und Perfluoropolyether (PFPE) als Gleitmittelkomponenten eingesetzt, obwohl beispielsweise die PFC seit Jahren sehr kritisch beurteilt werden.

Eine konsequente Anwendung von arbeitshygienischen und ökologischen Kriterien müsste viele der heute gebräuchlichen Gleitmittel für den Breitensport beschränken oder gar ausschliessen, wie das beispielsweise für die Perfluoroctansäure (PFOS und PFOA) und deren Salze sowie andere per- und polyfluorierte Alkylverbindungen (PFAS) aktuell der Fall ist. Auch andere Substanzen, die z.B. in der ECHA-Kandidaten-Liste der "Substances of Very High Concern" (SVHC) stehen, sollten angesichts ihrer erkannten Gefährdungen nicht verwendet werden. Leider fehlt es aber bei einer Vielzahl von gegenwärtig verwendeten Gleitmitteln an experimentellen Substanzdaten, um verlässliche arbeitshygienische und/oder ökologische Beurteilungen vornehmen zu können.

Durch die massenhafte Verwendung von traditionellen Skiwachsen und anderen modernen Gleitmitteln im Leistungs- und Freizeitsport und dem damit verbundenen Eintrag derselben in die Umwelt werden ökologische, aber auch arbeitshygienische Kriterien immer bedeutungsvoller. Das betrifft nicht nur die Herstellung und die Anwendung der Gleitmittel, sondern auch deren Abfälle, den Abrieb, die Rückstände und die Gleitmittel-Abbauprodukte.

Ein neues, arbeitshygienisch wie ökologisch weitgehend unbedenkliches Gleitmittel für Wintersportgeräte, wurde in WO 2019145282 offenbart. Es basiert auf dem natürlich vorkommenden Farbstoff Indigo und davon abgeleiteten Derivaten als alleiniger oder überwiegender, gleitwirksamer Gleitmittelkomponente.

Aufgabe der vorliegenden Erfindung ist es, ein alternatives Gleitmittel bereit zu stellen, das die Reibung der Gleitflächen von verschiedenen technischen Gegenständen und Geräten, insbesondere von Wintersport- und Wassersportgeräten, auf Eis, Schnee und/oder Wasser verringern kann und ökologisch und arbeitshygienisch weitgehend unkritisch ist.

### BESCHREIBUNG DER ERFINDUNG

Als erfindungsgemässes "Gleitmittel" wird im Nachfolgenden eine Gleitmittelzusammensetzung verstanden, die mindestens eine gleitwirksame, erfindungsgemässe Gleitmittelkomponente, oder eine Mischung erfindungsgemässer Gleitmittelkoponenten, in einem Mengenanteil von mindestens 5 Gewichtsprozent (abgekürzt: Gew.%), vorzugsweise mindestens 30 Gew.% und besonders bevorzugt mindestens 70 Gew..% aller gleitwirksamen Komponenten der Gleitmittelzusammensetzung, sowie gegebenenfalls weitere gleitwirksame und/oder nicht-gleitwirksame Zusätze enthält.

Unter "gleitwirksam" wird die Eigenschaft einer Gleitmittelkomponente verstanden, einen direkten und unmittelbaren Beitrag zur Senkung der Gleitreibung zwischen der Gleitfläche eines auf einem Untergrund aus Schnee, Eis und/oder Wasser gleitenden Gegenstandes und der dabei kontaktierten Kontaktfläche des Untergrunds zu leisten. Dem zu Folge sind nicht-gleitwirksame Komponenten einer Gleitmittelzusammensetzung solche, die keinen Beitrag zur Reibungsminderung zwischen Gleitfläche und Untergrund leisten. Zu letzteren gehören neben allfälligen Trägermaterialien, inbesondere Lösungsmitteln, für flüssige Gleitmittelzusammensetzungen vor allem Hilfsstoffe, die typischerweise nur in geringen Mengen zugesetzt werden wie beispielsweise Coatings, Farbstoffe, Füllstoffe, Duftstoffe, Emulgatoren, Suspensionshilfsstoffe.

Unter einer "gleitwirksamen Menge" wird jene Menge einer Gleitmittelkomponente in einer Gleitmittelzusammensetzung verstanden, die erforderlich oder ausreichend ist, um eine messbare Verminderung der Gleitreibung einer erfindungsgemäss behandelten Gleitfläche gegenüber einer unbehandelten Gleitfläche derselben Art, in Bezug auf eine gegebene Kontaktfläche, zu bewirken.

Im Regelfall sind die Laufflächen von Wintersportgeräten und von manchen Wassersportgeräten mit einem hydrophoben Belag ausgestattet, der die Lauffläche des jeweiligen Sportgerätes bedeckt und zumeist bestimmte dreidimensionale Strukturen an der Oberfläche aufweist. Bei Geräten und Gegenständen, wo ein derartiger Belag vorhanden ist, gilt die freie Oberfläche des Belages als "Gleitfläche" im Sinne der vorliegenden Erfindung. Wo ein derartiger Belag nicht vorhanden ist, gilt die bei üblicher Benutzung mit dem Untergrund in Kontakt tretende Lauffläche des Gerätes oder Gegenstandes als Gleitfläche im Sinne der Erfindung.

Die erfindungsgemässen Gleitmittel können grundsätzlich sowohl auf die als Gleitfläche fungierende Oberfläche des Belags des Gerätes oder Gegenstandes aufgebracht, als auch in den Belag eingearbeitet werden, beispielsweise im Zuge der Herstellung des jeweiligen Gerätes oder Gegenstandes. Bei Geräten und Gegenständen, insbesondere Winter- oder Wassersportgeräten, die keinen Laufflächenbelag aufweisen, können die erfindungsgemässen Gleitmittel auch direkt auf die Lauffläche, die in diesen Fällen dann als Gleitfläche fungiert, aufgebracht werden.

Für die Anwendung der vorliegenden Erfindung besonders geeignete Geräte und Gegenstände, welche Gleitflächen aufweisen, die bei bestimmungsgemässer Anwendung mit einer Kontaktfläche aus Schnee, Eis und/oder Wasser oder Wasserfilm in Kontakt treten, zählen vor allem Wintersportgeräte wie z.B. Alpinski, Langlaufski, Sprungski, Snowboard, Skibob, Rodel, Schlitten, Skeleton, und Bob. Daneben aber auch Wassersportgeräte wie z.B. Surfbretter, Standupbretter, Wasserskis, Kanus, Kajaks und Boote aller Art. Ebenso Gegenstände und Geräte, die nicht notwendigerweise für den sportlichen Einsatz bestimmt sind, wie z.B. sonstige Schneeschlitten, insbesondere Pferdeschlitten, aber auch Gleiter und Kufen für Flugzeuge, die auf Schnee oder Wasser starten und landen können, und natürlich Fracht- und Passagierschiffe bzw. deren Schiffsrümpfe, jeder Art und Grösse. Bei letzteren geht es weniger um einen Geschwindigkeitsgewinn durch Reibungsminderung als vielmehr um substanzielle Energieeinsparungen dank einer Beschichtung mit erfindungsgemässen Gleitmitteln auf der Basis von Chinacridonen.

Zu den weiteren technischen Anwendungen der erfindungsgemässen Gleitmittel zählt beispielsweise der Einsatz zur Beschichtung von Schneepflügen, Schneefräsen, Schneeschaufeln aller Art, und Schneebohrern, aber auch die Beschichtung von Wasserrohren, Wasserleitungen und wasserführenden Kanälen aller Art, sowie generell der Einsatz zur Beschichtung von Gleitflächen bestehender oder zukünftiger Gegenstände, die bei bestimmungsgemässer Verwendung mit Schnee, Eis und/oder Wasser in Kontakt stehen und bei denen eine Reibungsminderung zwischen Gleitfläche und Kontaktfläche einen zeitlichen, energetischen und/oder finanziellen Vorteil bringt.

Je nach Bedarf und Anwendungsform können erfindungsgemässe Gleitmittelkomponenten auch in einer Flüssigkeit gelöst, dispergiert, emulgiert, als Paste oder in Form von Mischungen, insbesondere als Mischung mit Wachsen, kalt oder heiss auf Gleitflächen aufgetragen, und/oder in die Strukturen von Gleitflächen-Belägen der jeweiligen Geräte eingearbeitet werden.

Erfindungsgemäss wird ein verbessertes Gleiten einer Gleitfläche auf einem Untergrund aus Eis, Schnee und/oder Wasser dadurch erzielt, dass die Gleitreibung mit Farbpigmenten aus der Gruppe der Chinacridone (synonym: Quinacridone, QAC) stark verringert wird. Chinacridon-Pigmente (QACs) sind eine Gruppe organischer Pigmente, die sich von der Grundstruktur des Chinacridons (IUPAC-Name: 5,12-Dihydro-quino[2,3-b]acridine-7,14-dione) ableiten.

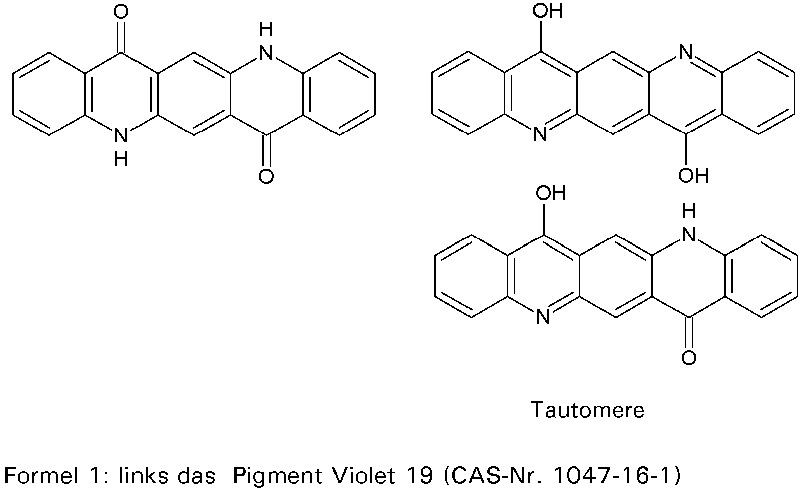

| | |
|---|---|
| IUPAC-Name: | 5,7,12,14-tetrahydro-5,12-diazapentacene-7,14-dione |
| EC-Name | 5,12-dihydroquinolino[2,3-b]acridine-7,14-dione (InChIKey: NRCMAYZCPIVABH-UHFFFAOYSA-N); |

rechts die beiden Tautomere davon. Auch das cis-Isomer des Pigments Violet 19 und seine beiden Tautomere gehören zu dieser ersten Gruppe der erfindungsgemäss einsetzbaren Chinacridone.

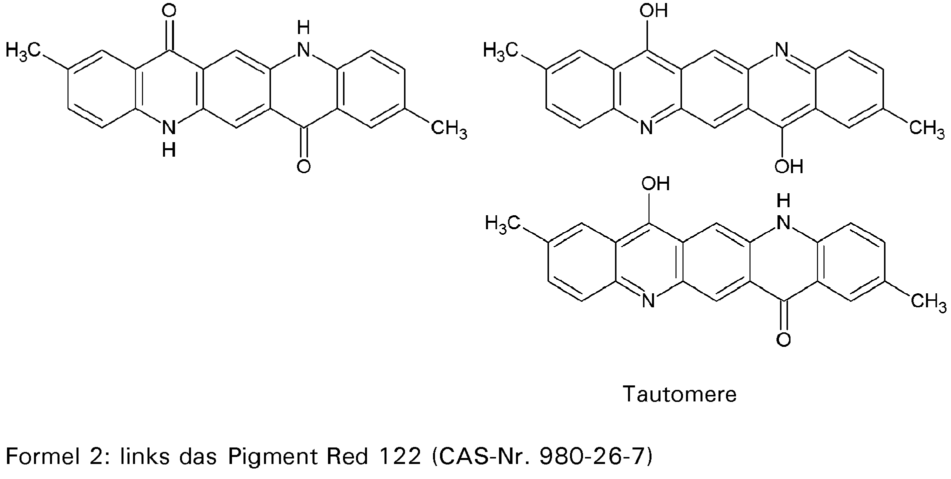

| | |
|---|---|
| IUPAC: | 2,9-dimethyl-5,7,12,14-tetrahydro-5,12-diazapentacene-7,14-dione |
| EC: | 5,12-dihydro-2,9-dimethylquino[2,3-b]acridine-7,14-dione |
| | (InChIKey: TXWSZJSDZKWQAU-UHFFFAOYSA-N); |

rechts die beiden Tautomere davon. Auch das cis-Isomer des Pigments Red 122 und seine beiden Tautomere gehören zu dieser zweiten Gruppe der erfindungsgemäss einsetzbaren Chinacridone.

Die Chinacridone können als cis- oder trans-Isomere, lineare oder angulare Isomere, und gleichzeitig als Tautomere vorliegen. Die erfindungsgemässen, Chinacridon-Verbindungen (QACs) umfassen insbesondere die ChinacridonMoleküle gemäss Formel 1 und Formel 2, sowie dichlor-substituierte Chinacridon-Derivate wie z.B.:
- die Pigmente Red 202 (CAS-Nr. 3089-17-6, 61932-63-6, 68859-50-7), 2,9-dichloro-5,12-dihydroquino[2,3-b]acridine-7,14-dione;
- das Pigment Red 209 (CAS-Nr. 3573-01-1);
- ein Gemisch aus 1,8-dichloro-5,7,12,14-tetrahydro-5,12-diazapentacene-7,14-dione mit 1,9-dichloro-5,7,12,14-tetrahydro-5,12-diazapentacene-7,14-dione und 2,9-dichloro-5,7,12,14-tetrahydro-5,12-diazapentacene-7,14-dione; oder
- ein Gemisch aus 1,8-dichloro-5,7,12,14-tetrahydro-5,12-diazapentacene-7,14-dione mit 1,9-dichloro-5,7,12,14-tetrahydro-5,12-diazapentacene-7,14-dione und 3,10-dichloro-5,7,12,14-tetrahydro-5,12-diazapentacene-7,14-dione);
- Pigment Red 207 (CAS-Nr. 71819-77-7),
- Pigment Red 206 (CAS-Nr. 71819-77-6); und
- Pigment Red 210 (CAS-Nr. 61932-63-6), 2,9-dichloro-5,7,12,14-tetrahydro-2-diazapentacene-7,14-dione.

"CAS-Nr." ist die Abkürzung für "Chemical Abstracts Service Number" und dient zur Identifikation der chemischen Verbindungen.
"InChlKey" (International Chemical Identifier Key) ist der Liniencode der IUPAC, der die eindeutige Identifizierung chemischer Strukturen ermöglicht, wobei tautomere Strukturen nicht unterschieden werden. Die für die vorliegende Erfindung geeigneten, gleitwirksamen Chinacridon-Verbindungen können bei gleichen CAS-Nummern in unterschiedlichen Tautomerien vorliegen und schliessen diese mit ein.

Chinacridone werden als Farbstoff-Pigmente z.B. in Anstrichfarben, Lacken aber auch in Tonern verwendet. Sie können selbstorganisierende, supramolekulare organische Halbleiter bilden und sind auch als triboelektrische Substanzen bekannt. Der Einbau des Farbstoffs Chinacridon in das Polymer Polypropylen ist bekannt, ebenso wie dessen Verwendung in Verfahren zum Färben textiler Fasern, Geflechte und Laminatmaterialien, speziell auch des Materials Ultra-High Molecular Weight Polyethylene (UHMWPE). WO2016151409A1 beschreibt beispielsweise die guten mechanischen Eigenschaften der mit Chinacridon gefärbten Polymere. Dabei bilden die Chinacridone Schuppen, die sich in Laufrichtung der Polymerfasern ausrichten, ein Effekt, der auch beim händischen Auftragen von Chinacridon-Pulver mithilfe eines Filzes auf z.B. einen Skibelag spürbar ist.

Charakteristisch für Chinacridon-Pigmente ist die sehr gute Witterungs-, Licht- und Oxidationsbeständigkeit, eine hohe Farbstärke, eine herausragende chemische Beständigkeit, sowie eine hohe Temperaturstabilität bis 450°C bei gleichzeitig geringer Toxizität und Ökotoxizität. Typisch ist allerdings auch das mässige Deckvermögen, verbunden mit einer hohen Transparenz und einer schlechten Dispergierbarkeit.

Wichtig für die Anwendung in Gleitmitteln für Gleitflächen auf Eis, Schnee oder Wasser ist die chemische Stabilität und die Bildung von harten, hydrophoben, selbstorganisierenden supramolekularen Strukturen über die intermolekularen Wasserstoffbrückenbindungen. Für die Ausbildung von Schichtstrukturen und die erwünschte Bindung an die vorgesehenen Gleitflächen ist die Vielzahl der London-Kräfte der π-Bindungen von supramolekularen polyaromatischen Chinacridon-Bändern von Bedeutung. Die Chinacridon-Stapel werden durch π-π-Bindungen zusammen gehalten.

Durch eine zumindest geringfügige, elektrische Leitfähigkeit im Kontaktbereich Gleitfläche-Wasser/Schnee/Eis, wird die durch die Gleitreibung entstehende, elektrostatische Aufladung verhindert oder zumindest wesentlich vermindert und somit auch das Anhaften von Schmutzpartikeln an der Gleitfläche weitgehend unterbunden. Die Härte der Chinacridon-Beschichtung verringert zudem das Eindringen von Schmutzpartikeln, welche ansonsten die Gleitreibung erhöhen könnten.

Die schwere Löslichkeit der Chinacridone in den meisten Lösungsmitteln, ausser in Trifluoressigsäure, konzentrierter Schwefelsäure oder konzentrierter Phosphorsäure, hat allerdings den Vorteil, dass eine wesentliche Aufnahme von Chinacridon-Verbindungen in den menschlichen Körper nicht stattfindet und diese Verbindungen daher als nur sehr gering toxisch eingestuft werden.

Die erfindungsgemäss geeigneten Chinacridone sind durch folgende allgemeine chemische Formeln dargestellt: 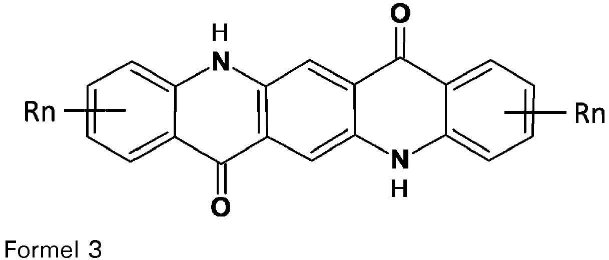 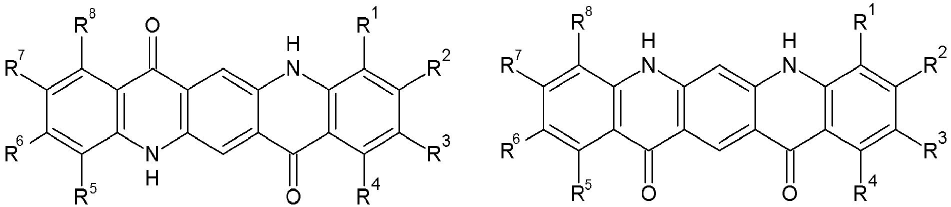 wobei die Substituenten R1 bis R8 Wasserstoff-, Fluor-, Chlor-, oder Bromatome oder C1-C2 Alkylreste (-CH₃ oder -CH₂-CH₃) sein können.

Für einige der vorgesehenen Verwendungszwecke sind die an R1/R5, R2/R6, R3/R7 und R4/R8 disubstituierten Derivate sowohl als cis- als auch als trans-Isomere (Z- und E-Isomere) in beta- oder gamma-Modifikation, einschliesslich deren Tautomere, besonders vorteilhaft.

Zusätzlich zu allen Grundstrukturen von Chinacridon und dessen Derivaten schliessen die erfindungsgemäss als Gleitmittelkomponenten verwendbaren Chinacridon-Verbindungen auch die - durch die Umgebung beeinflussbaren - tautomeren Strukturen, sowie die thermisch leicht umwandelbaren, unterschiedlichen Kristallmodifikationen alpha-I, alpha-II, beta und gamma (siehe z.B. US2969366, US3007930, US3009916) mit ein.

Die tautomeren Strukturen sind deshalb wichtig, weil sich die Moleküle über Elektronen- und Wasserstoffverschiebungen in einem gewissen Bereich an die Polarität der Umgebung anpassen können. Das hat eine besondere Bedeutung, wenn Chinacridon auf zwei chemisch unterschiedliche Oberflächen trifft, was im vorliegenden Fall mit den erfindungsgemäss präparierten Gleitflächen der diversen Gegenstände und Geräte auf der einen Seite und den Kontaktflächen des Untergrunds aus Schnee, Eis und/oder Wasser bzw. Wasserfilm auf der anderen Seite stets gegeben ist.

Aufgrund noch nicht verfügbarer experimenteller Daten zu den physikalischen, chemischen, toxikologischen und ökologischen Eigenschaften der erfindungsgemäss als Gleitmittelkomponenten einsetzbaren Chinacridon-Verbindungen, wurden diese Eigenschaften mit dem etablierten "Quantitative-Structure-Activity-Relationship"- Verfahren (QSAR) als abgeschätzte Werte ermittelt.

Die polaren Gruppen > C = O, > N-H, bzw. -O-H und ≥N sind nicht nur für die autonome Ausbildung der supramolekularen Gleitflächen verantwortlich, sie ermöglichen auch den für die Ökologie so wichtigen, biologischen Abbau.

Je nach Bedarf und Verwendungszweck kann das erfindungsgemässe Gleitmittel eine einzelne Chinacridon-Verbindung als Gleitmittelkomponente in einer gleitwirksamen Menge enthalten oder ein Gemisch von zwei oder mehr verschiedenen Chinacridon-Verbindungen. Durch die Verwendung von zwei oder mehr unterschiedlichen Chinacridonen oder Chinacridonderivaten, einschliesslich deren halogenierter Derivate, können die Eigenschaften des Gleitmittels weiter verfeinert und auf aktuelle Untergrund-, Witterungs- und Temperaturbedingungen bestmöglich abgestimmt werden.

Ohne durch die Theorie gebunden zu sein, könnten die hervorragenden Gleiteigenschaften der erfindungsgemäss einsetzbaren Chinacridon-Verbindungen darauf zurück zu führen sein, dass diese Moleküle zum Einen eine sehr geringe Wasserlöslichkeit zeigen und zum Anderen eine planare, sich in einer Ebene erstreckende, Struktur aufweisen. Diese planare Molekülstruktur ermöglicht es den Chinacridonen, Schichten auszubilden, die sich supramolekular mit Reibung oder Druck durch self-assembly organisieren. Der Aufbau dieser Schicht-Struktur erfolgt lateral durch Wasserstoffbrückenbindungen (> C = O, > N-H) und axial durch starke π-π-Wechseiwirkungen. Dabei ist jedes Chinacridon über Wasserstoffbrücken mit zwei weiteren Chinacridonen verbunden, wobei sich durch die intermolekulare Verlinkung harte Schuppen ausbilden, die die Hydrophobizität gegenüber Eis, Schnee und Wasser sogar noch steigern.

Das Auftragen eines erfindungsgemässen Gleitmittels auf eine Gleitfläche kann in Form einer Beschichtung erfolgen, die so dünn ist, dass sie allenfalls vorhandene, dreidimensionale Belagsstrukturen der Gleitfläche praktisch verlustfrei abdeckt. Die spezifischen elektrostatischen Eigenschaften und die Härte der erfindungsgemässen Gleitmittelkomponenten vermindern zudem die Aufnahme von Verunreinigungen als Folge des Gleitens auf verschmutztem Eis, Schnee oder Wasser bzw. Wasserfilm. Somit werden mit diesen Chinacridon-Molekülen nicht nur die Gleiteigenschaften der verschiedenen Geräte und Gegenstände verbessert, sondern durch den - gegenüber herkömmlichen Gleitmitteln auf Paraffinbasis - geringeren Abrieb, auch deren Gebrauchsdauer verlängert. Dass die intermolekularen Molekül-Cluster planar bleiben, kann mikroskopisch als Oberflächenstruktur nachgewiesen werden.

Durch die Einbettung der Chinacridonmoleküle in Polymere, Paraffine, Wachse oder andere, als Gleitmittel oder Gleitmittelkomponenten geeignete, hydrophobe Massen, kann die Festigkeit dieser hydrophoben Massen erhöht und dadurch deren Qualität gesteigert werden. Insbesondere kann auf diese Weise deren Eignung zur Ausbildung stabiler, abriebfester Gleitschichten verbessert werden.

Die erfindungsgemässen Gleitmittel auf Chinacridonbasis können grundsätzlich sowohl auf die als Gleitfläche fungierende, belagsfreie Lauffläche, beispielsweise eines Winter-oder Wassersportgerätes, aufgetragen oder - bei Vorhandensein eines hydrophoben Belages - sowohl auf die Oberfläche des Belags als auch in den Belag eingebracht, respektive eingearbeitet werden. Beispielsweise kann ein in Form eines Festkörpers oder als Pulver vorliegendes, erfindungsgemässes Gleitmittel auf die Gleitfläche aufgerieben, aufgeschmolzen, aufsublimiert oder anderweitig aufgetragen werden. Die erfindungsgemässen Gleitmittelkomponenten können aber auch in einer Flüssigkeit gelöst, dispergiert, emulgiert, als Paste oder als Mischungen mit Wachsen, kalt oder heiss auf eine belagsfreie Gleitfläche oder einen Belag aufgetragen und/oder in den Belag eingearbeitet werden.

### KURZE BESCHREIBUNG DER ABBILDUNGEN

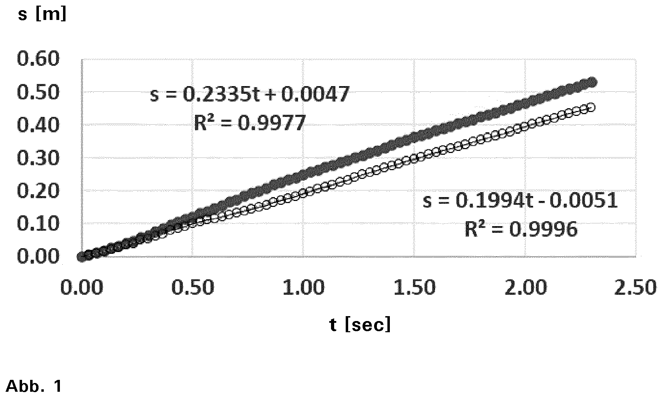

Abb. 1: zeigt die Beschleunigung von Wassertropfen à 50µl auf einem Langlaufski mit UHMWEPE-Graphit Belag vor und nach Aufbringen eines erfindungsgemässen Gleitmittels auf Chinacridon-Basis, bei einem Neigungs-winkel des Skis von 30 Grad und einer Temperatur von 20°C; X-Achse = Zeit in Sekunden; Y-Achse = zurückgelegte Wegstrecke der Tropfen in Metern; untere Kurve (leere Kreise) = vor der Behandlung, obere Kurve (gefüllte Kreise) = nach der Behandlung mit Gleitmittel; Werte sind Durchschnittswerte aus 10 Versuchen.

Die nachfolgenden Beispiele dienen der weiteren Verdeutlichung der Erfindung.

### BEISPIEL 1: Gleittest von Wassertropfen auf einem präparierten Ski

Eine Dispersion von hochgereinigtem Chinacridon (> 99% Gew.; CAS No: 980-26-7) wird mit einem Schwamm auf die wachsfreie, mit einer Stahlbürste gebürstete UHMWPE-Graphit-Oberfläche eines Langlauf-Skis mit mittlerem Schliff aufgetragen und mit einem Filz eingerieben. Dieser Vorgang wird solange wiederholt, bis sich eine fleckenfreie Schicht gebildet hat. Danach wird die Oberfläche mit einem Bügeleisen mit einer Temperatur von 110 °C langsam von der Ski-Spitze zum Ski-Ende hin, erwärmt. Schliesslich wird die dabei entstehende, dünne Schicht mit einer Rosshaarbürste poliert.

Die so behandelte Oberfläche ist sehr glatt und lässt Wassertropfen von 50 µl bei einem Neigungs-Winkel von 30° und einer Temperatur von 20°C mit einer Geschwindigkeit von 0.2335 m/s gleiten. Auf einer unbehandelten Oberfläche desselben Skis bewegen sich die Wassertropfen bei gleichen Bedingungen mit einer Geschwindigkeit von lediglich 0.1994m/s. Das Präparieren des Skis mit dem erfindungsgemässen Gleitmittel bewirkt also eine Geschwindigkeitssteigerung von ca. 17%.

Die Regressionsgeraden der beiden Messkurven in Abb. 1 sind als Formel für eine gleichförmige Bewegung (s = v*t + s₀ ; s = Strecke, v = Geschwindigkeit, t = Zeit, s₀ = Anfangsweg) dargestellt. In der Abb. 1 ist R² das Bestimmtheitsmass, das in der Statistik eine Kennzahl zur Beurteilung der Anpassungsgüte einer Regression ist und beschreibt wie gut Messwerte zum Modell passen.

### BEISPIEL 2: Abriebfestigkeit

Einer wie in Beispiel 1 präparierten Belagsoberfläche wird mit einem Handstrukturgerät eine 0.1 mm tiefe, lineare Struktur mit geringem Druck dreimal von der Ski-Spitze zum Ski-Ende eingepresst. Sodann wird die Chinacridon-Dispersion nochmals aufgetragen, eingerieben, mit dem Bügeleisen erwärmt und poliert. Die so gebildete Schicht zeigt selbst bei sehr langer Beanspruchung über eine Distanz von mehr als 70 km, bei aggressivem, trockenem Schnee und ca. -10 °C Lufttemperatur, einen so geringen Abrieb, dass keine hellen Stellen sichtbar sind.

### BEISPIEL 3: Präparieren eines Sprungskis mit einer Mischung aus Chinacridon und Wachs

100 Gramm eines Paraffin-Wachses (z.B. Sasolwax 6403) werden bei einer Temperatur von 100 °C geschmolzen. In diese Schmelze wird dieselbe Menge (100 g) hochgereinigtes Chinacridon (Pigment Violet 19, CAS-Nr. 1047-16-1) eingerührt, bis die Mischung weitestgehend homogen ist. Die Mischung wird mit einem Wachseisen bei ca. 110-120 °C auf die Belagsoberfläche aufgetropft und gleichmässig verteilt, wie das beim Heisswachsen von Skis oder Snowboards üblich ist. Die weiteren Arbeitsschritte, wie z.B. mit der Klinge "Abziehen" ( = glatt hobeln, um Gleitmittel-Überschüsse und Unebenheiten zu entfernen), Ausbürsten und Polieren, entsprechen dem bekannten Vorgehen bei Verwendung von Heisswachsen.

### BEISPIEL 4: Präparieren eines Skis mittels Gleitmittelpulver

Als Gleitmittel wird im Handel erhältliches Pigment Red 122 (CAS-Nr. 980-26-7) als einzige Gleitmittelkomponente, ohne weitere Zusätze, verwendet.

Pigment Red 122 wird als Pulver auf der Oberfläche eines ungewachsten oder gewachsten Belags eines Alpinskis gleichmässig verteilt und mit einem Korkblock solange in Laufrichtung kräftig eingerieben, bis die Oberfläche ganz mit einer dünnen Schicht bedeckt ist. Danach wird das Chinacridon Pigment Red 122 mit einer Kupferbürste so verteilt, dass es auch die Vertiefungen der BelagsStrukturen ganz bedeckt. Dieser Vorgang kann mehrmals wiederholt werden. Wahlweise kann im Anschluss zusätzlich noch ein wenig Indigo eingerieben werden. Abschliessend wird die Oberfläche mit einer Polierbürste, z.B. aus Rosshaar, poliert.

## Patentansprüche

1. Verfahren zum Vermindern der Reibung einer Gleitfläche eines bei bestimmungsgemässer Anwendung mit einer Kontaktfläche aus Schnee, Eis und/oder Wasser in Kontakt stehenden Gerätes oder Gegenstandes gegenüber einer Kontaktfläche aus Schnee, Eis, Wasser, oder einer Mischung davon, **dadurch gekennzeichnet, dass** ein Gleitmittel, welches mindestens eine Chinacridonverbindung als gleitwirksame Gleitmittelkomponente enthält, in fester oder flüssiger Form auf die Gleitfläche aufgebracht oder - sofern die Gleitfläche als hydrophober Belag ausgebildet ist - auf die Gleitfläche aufgetragen und/oder in die Gleitfläche eingebracht wird.

2. Verfahren nach Anspruch 1, **dadurch gekennzeichnet, dass** die Chinacridon-Verbindung folgende Strukturformel aufweist: 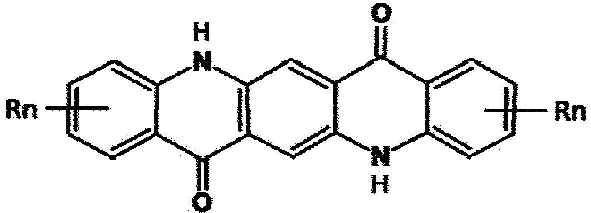 wobei die Substituenten Rn Wasserstoff-, Fluor-, Chlor-, oder Bromatome oder C1-C2-Alkylreste (-CH₃ oder -CH₂-CH₃) sein können.

3. Verfahren nach Anspruch 1, **dadurch gekennzeichnet, dass** die Chinacridon-Verbindung folgende Strukturformel aufweist: 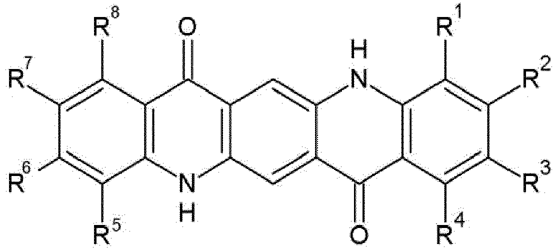
wobei die Substituenten R¹ bis R⁸ Wasserstoff-, Fluor-, Chlor-, oder Bromatome oder C1-C2 -Alkylreste (-CH₃ oder -CH₂-CH₃) sind, und
wobei die Chinacridon-Verbindung vorzugsweise in Form von an R¹ und R⁵, R² und R⁶, R³ und R⁷, und/oder R⁴ und R⁸ disubstituierten Derivaten als cis- oder trans-Isomere (Z- und E-Isomere) in beta- oder gamma-Modifikation, sowie in Form ihrer Tautomere vorliegt.

4. Verfahren nach einem der Ansprüche 1 bis 3, **dadurch gekennzeichnet, dass** die Chinacridon-Verbindung methylierte Derivate, halogenierte Derivate, cis-trans Isomere, Tautomere, sowie Varianten der Chinacridone und deren Derivate , einschliesslich der Isomere und Tautomere, in den Kristallstrukturen alpha I, alpha II, beta, und gamma umfasst.

5. Verfahren nach einem der Ansprüche 1 bis 4, **dadurch gekennzeichnet, dass** das Gleitmittel mindestens eine gleitwirksame Wachskomponente und/oder mindestens einen nicht-gleitwirksamen Zusatz, vorzugsweise aus der Gruppe Trägermaterialien für flüssige Gleitmittelzusammensetzungen, Lösungsmittel, Coatings, Farbstoffe, Füllstoffe, Duftstoffe, Emulgatoren, Suspensionshilfsstoffe, enthält.

6. Verfahren nach Anspruch 5, **dadurch gekennzeichnet, dass** der Anteil der Chinacridon-Verbindungen am Gleitmittel in einem Bereich von 1 Gew.% bis 50 Gew%, typischerweise von 5 bis 95 Gew.%, und vorzugsweise von 20 bis 100 Gew.% liegt.

7. Verfahren nach einem der Ansprüche 5 oder 6, **dadurch gekennzeichnet, dass** der Anteil der Chinacridon-Verbindungen an der Gesamtheit der gleitwirksamen Gleitmittelkomponenten des Gleitmittels in einem Bereich von 5 - 100 Gew%, vorzugsweise von 30 - 100 Gew%, und insbesondere von 70 - 100 Gew% liegt.

8. Verfahren nach einem der Ansprüche 1 bis 7, **dadurch gekennzeichnet, dass** das Gleitmittel als Feststoff, Pulver, Paste, Schmelze, Lösung, Dispersion oder Emulsion auf die Gleitfläche aufgetragen oder in die Gleitfläche eingebracht wird.

9. Verwendung mindestens einer Chinacridon-Verbindung als gleitwirksame Gleitmittelkomponente in einem Gleitmittel zum Präparieren einer Gleitfläche eines bei bestimmungsgemässer Anwendung mit einer Kontaktfläche aus Schnee, Eis und/oder Wasser in Kontakt stehenden Gegenstandes oder Gerätes, zur Verminderung der Reibung zwischen der Gleitfläche und einer Kontaktfläche aus Schnee, Eis, Wasser, oder einer Mischung davon.

10. Verwendung nach Anspruch 9, wobei der Gegenstand oder das Gerät ausgewählt sind aus der Gruppe der
- Wintersportgeräte umfassend Alpinski, Langlaufski, Sprungski, Snowboard, Skibob, Rodel, Schlitten, Skeleton, und Bob;
- aus der Gruppe der Wassersportgeräte umfassend Surfbretter, StandupBretter, Wasserskis, Kanus, Kajaks, und Boote aller Art; und aus der Gruppe
- sonstiger Gegenstände und Geräte, umfassend Schneeschlitten, insbesondere Pferdeschlitten, Gleiter und Kufen für Flugzeuge, die auf Schnee oder Wasser starten und landen können, Schneepflüge, Schneefräsen, Schneeschaufeln aller Art, Schneebohrer, Wasserrohre, Wasserleitungen, wasserführende Kanäle, und
- Schiffsrümpfe von Fracht- und Passagierschiffen.

11. Verwendung nach Anspruch 9, wobei das Gleitmittel als Feststoff, Pulver, Paste, Schmelze, Lösung, Dispersion oder Emulsion vorliegt und eine gleitwirksame Menge, vorzugsweise 1 - 50 Gew%, typischerweise 5 - 95 Gew.%, und vorzugsweise 20 - 99 Gew.% mindestens einer Chinacridon-Verbindung als Gleitmittelkomponente enthält.

12. Verwendung nach Anspruch 9, wobei die Chinacridon-Verbindung folgende Strukturformel aufweist: 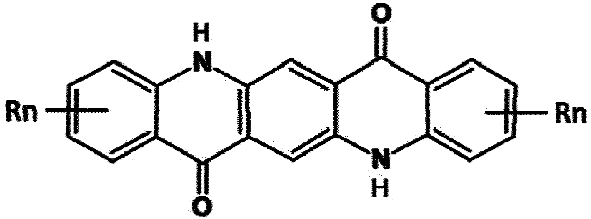 wobei die Substituenten Rn Wasserstoff-, Fluor-, Chlor-, oder Bromatome oder C1-C2-Alkylreste (-CH₃ oder -CH₂-CH₃) sein können.

13. Verwendung nach Anspruch 9, wobei die Chinacridon-Verbindung folgende Strukturformel aufweist: 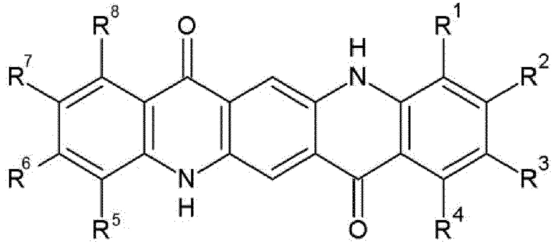
wobei die Substituenten R¹ bis R⁸ Wasserstoff-, Fluor-, Chlor-, oder Bromatome oder C1-C2 -Alkylreste (-CH₃ oder -CH₂-CH₃) sein können, und
wobei an R¹ und R⁵, R² und R⁶, R³ und R⁷, sowie R⁴ und R⁸ disubstituierte Derivate vorzugsweise als cis- oder trans-Isomere (Z- und E-Isomere) in beta- oder gamma-Modifikation sowie in Form ihrer Tautomere vorliegen.

14. Verwendung nach einem der Ansprüche 9 bis 13, wobei die Chinacridon-Verbindung methylierte Derivate, halogenierte Derivate, cis-trans Isomere, Tautomere, sowie Varianten der Chinacridone und deren Derivate, einschliesslich deren Isomere und Tautomere, in den Kristallstrukturen alpha I, alpha II, beta, und gamma, umfasst.

## Claims

1. Method for reducing the friction of a sliding surface of a device or object which, when used as intended, is in contact with a contact surface of snow, ice and/or water with respect to a contact surface of snow, ice, water or a mixture thereof, **characterized in that** a lubricant which contains at least one quinacridone compound as a lubricant component with a sliding effect is applied to the sliding surface in solid or liquid form or - if the sliding surface is designed as a hydrophobic coating - is applied to the sliding surface and/or introduced into the sliding surface.

2. The method according to claim 1, **characterized in that** the quinacridone compound has the following structural formula:
wherein the substituents Rn may be hydrogen, fluorine, chlorine or bromine atoms or C1-C2 alkyl radicals (-CH3 or -CH2-CH3).

3. The method according to claim 1, **characterized in that** the quinacridone compound has the following structural formula: 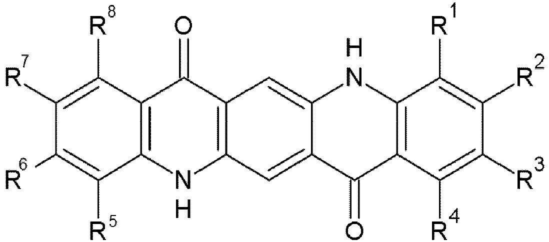
where the substituents R1 to R8 are hydrogen, fluorine, chlorine or bromine atoms or C1-C2-alkyl radicals (-CH3 or -CH2-CH3), and
wherein the quinacridone compound is preferably present in the form of derivatives disubstituted at R1 and R5, R2 and R6, R3 and R7, and/or R4 and R8 as cis- or trans-isomers (Z- and E-isomers) in beta- or gamma-modification, and in the form of their tautomers.

4. Method according to one of claims 1 to 3, **characterized in that** the quinacridone compound comprises methylated derivatives, halogenated derivatives, cis-trans isomers, tautomers, and variants of the quinacridones and their derivatives, including the isomers and tautomers, in the crystal structures alpha I, alpha II, beta, and gamma.

5. Method according to one of claims 1 to 4, **characterized in that** the lubricant contains at least one lubricating wax component and/or at least one non-lubricating additive, preferably from the group consisting of carrier materials for liquid lubricant compositions, solvents, coatings, dyes, fillers, fragrances, emulsifiers and suspension aids.

6. Method according to claim 5, **characterized in that** the proportion of quinacridone compounds in the lubricant is in a range from 1% to 50% by weight, typically from 5% to 95% by weight, and preferably from 20% to 100% by weight.

7. Method according to one of claims 5 or 6, **characterized in that** the proportion of the quinacridone compounds in the total of lubricating components of the lubricant is in a range from 5 to 100% by weight, preferably from 30 to 100% by weight, and in particular from 70 to 100% by weight.

8. Method according to one of claims 1 to 7, **characterized in that** the lubricant is applied to the sliding surface or introduced into the sliding surface as a solid, powder, paste, melt, solution, dispersion or emulsion.

9. Use of at least one quinacridone compound as a lubricant component with a sliding effect in a lubricant for treating a sliding surface of an object or device which, when used as intended, is in contact with a contact surface made of snow, ice and/or water, in order to reduce the friction between the sliding surface and a contact surface made of snow, ice, water or a mixture thereof.

10. Use according to claim 9, wherein the object or device is selected from the group of
- winter sports equipment comprising alpine skis, cross-country skis, ski jump skis, snowboards, skibobs, toboggans, sledges, skeletons and bobsleds;
- from the group of water sports equipment comprising surfboards, stand-up boards, water skis, canoes, kayaks and boats of all kinds; and
- from the group of other objects and devices, including snow sledges, in particular horse-drawn sleighs, gliders and runners for aircraft that can take off and land on snow or water, snow plows, snow blowers, snow shovels of all kinds, snow drills, water pipes, water conduits, water-carrying channels, and
- ship hulls of freight and passenger ships.

11. Use according to claim 9, wherein the lubricant is in the form of a solid, powder, paste, melt, solution, dispersion or emulsion and contains a sliding-effective amount, preferably 1-50% by weight, typically 5-95% by weight, and preferably 20-99% by weight, of at least one quinacridone compound as a lubricant component.

12. The use according to claim 9, wherein the quinacridone compound has the following structural formula:
wherein the substituents Rn may be hydrogen, fluorine, chlorine or bromine atoms or C1-C2 alkyl radicals (-CH3 or -CH2-CH3).

13. The use according to claim 9, wherein the quinacridone compound has the following structural formula: 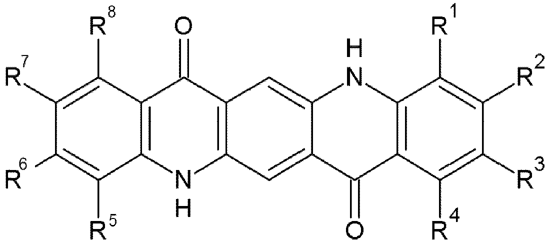
where the substituents R1 to R8 can be hydrogen, fluorine, chlorine or bromine atoms or C1-C2 alkyl radicals (-CH3 or -CH2-CH3), and
wherein derivatives disubstituted at R1 and R5, R2 and R6, R3 and R7, and R4 and R8 are preferably present as cis or trans isomers (Z and E isomers) in beta or gamma modification and in the form of their tautomers.

14. Use according to any one of claims 9 to 13, wherein the quinacridone compound comprises methylated derivatives, halogenated derivatives, cis-trans isomers, tautomers, and variants of the quinacridones and their derivatives, including their isomers and tautomers, in the crystal structures alpha I, alpha II, beta, and gamma.

## Revendications

1. Procédé pour réduire le frottement d'une surface de glissement d'un appareil ou d'un objet qui, lorsqu'il est utilisé comme prévu, est en contact avec une surface de contact constituée de neige, de glace et/ou d'eau, par rapport à une surface de contact constituée de neige, de glace, d'eau, ou un mélange de ceux-ci, **caractérisé en ce qu'**un lubrifiant, qui contient au moins un composé de quinacridone en tant que composant lubrifiant à effet glissant, est appliqué sous forme solide ou liquide sur la surface de glissement ou - si la surface de glissement est réalisée sous forme de revêtement hydrophobe - est appliqué sur la surface de glissement et/ou est introduit dans la surface de glissement.

2. Procédé selon la revendication 1, **caractérisé en ce que** le composé quinacridone répond à la formule développée suivante :
où les substituants Rn peuvent être des atomes d'hydrogène, de fluor, de chlore ou de brome ou des radicaux alkyles en C1-C2 (-CH3 ou -CH2-CH3).

3. Procédé selon la revendication 1, **caractérisé en ce que** le composé quinacridone répond à la formule développée suivante : 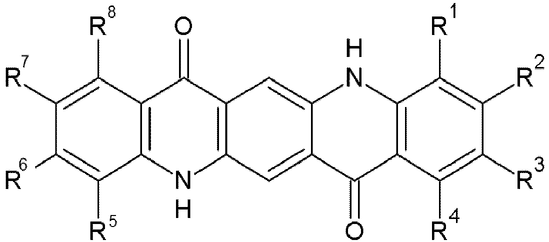
où les substituants R1 à R8 sont des atomes d'hydrogène, de fluor, de chlore ou de brome ou des radicaux alkyles en C1-C2 (-CH3 ou -CH2-CH3), et
où le composé quinacridone se présente de préférence sous forme de dérivés disubstitués sur R1 et R5, R2 et R6, R3 et R7, et/ou R4 et R8 en isomères cis ou trans (isomères Z et E) en modification bêta ou gamma, comme ainsi que sous la forme de leurs tautomères.

4. Procédé selon l'une quelconque des revendications 1 à 3, **caractérisé en ce que** le composé quinacridone contient des dérivés méthylés, des dérivés halogénés, des isomères cis-trans, des tautomères, et des variants des quinacridones et de leurs dérivés, y compris les isomères et tautomères, en les structures cristallines alpha I, alpha II, bêta et gamma.

5. Procédé selon l'une quelconque des revendications 1 à 4, **caractérisé en ce que** le lubrifiant contient au moins un composant de cire lubrifiante et/ou au moins un additif non lubrifiant, de préférence du groupe des matériaux supports pour compositions lubrifiantes liquides, solvants, revêtements, colorants, charges, parfums, émulsifiants, aides à la suspension.

6. Procédé selon la revendication 5, **caractérisé en ce que** la proportion des composés de quinacridone dans le lubrifiant se situe dans une plage de 1 % à 50% en poids, typiquement de 5 à 95% en poids, et de préférence de 20 à 100 % en poids..

7. Procédé selon l'une quelconque des revendications 5 ou 6, **caractérisé en ce que** la proportion de composés quinacridone dans le total des composants lubrifiants du lubrifiant se situe dans une plage de 5 à 100% en poids, de préférence de 30 à 100% en poids, et en particulier de 70 à 100% en poids.

8. Procédé selon l'une quelconque des revendications 1 à 7, **caractérisé en ce que** le lubrifiant est appliqué sur la surface de glissement ou introduit dans la surface de glissement sous forme solide, poudre, pâte, fondu, solution, dispersion ou émulsion.

9. Utilisation d'au moins un composé de quinacridone comme composant lubrifiant efficace dans un lubrifiant pour préparer une surface de glissement d'un objet ou d'un appareil qui, lorsqu'il est utilisé comme prévu, est en contact avec une surface de contact constituée de neige, de glace et/ ou de l'eau, pour réduire le frottement entre la surface de glissement et une surface de contact constituée de neige, de glace, d'eau ou d'un mélange de ceux-ci.

10. Utilisation selon la revendication 9, dans laquelle l'article ou l'appareil est choisi dans le groupe
- des équipements de sports d'hiver comprenant le ski alpin, le ski de fond, le ski de saut, le snowboard, le skibob, la luge, le traîneau, le skeleton et le bobsleigh;
- dans le groupe des équipements de sports nautiques comprenant les planches de surf, les planches de standup, les skis nautiques, les canoës, les kayaks et les bateaux de tous types; et
- dans le groupe des autres objets et équipements, comprenant les traîneaux à neige, notamment les traîneaux à chevaux, les planeurs et les patins pour avions pouvants décoller et atterrir sur la neige ou sur l'eau, les chasse-neige, les fraises à neige, les pelles à neige de tous types, les perce-neige, les tuyaux d'eau, les conduites d'eau, les canaux d'eau, et
- les coques de navires de marchandises et de passagers.

11. Utilisation selon la revendication 9, dans laquelle le lubrifiant se présente sous la forme d'un solide, d'une poudre, d'une pâte, d'une masse fondue, d'une solution, d'une dispersion ou d'une émulsion et contient une quantité efficace pour le glissement, de préférence de 1 à 50% en poids, typiquement de 5 à 95% en poids, et de préférence de 20 à 99% en poids d'au moins un composé de quinacridone comme composant lubrifiant.

12. Utilisation selon la revendication 9, dans laquelle le composé quinacridone a la formule structurelle suivante:
où les substituants Rn peuvent être des atomes d'hydrogène, de fluor, de chlore ou de brome ou des radicaux alkyles en C1-C2 (-CH3 ou -CH2-CH3).

13. Utilisation selon la revendication 9, dans laquelle le composé quinacridone a la formule structurelle suivante: 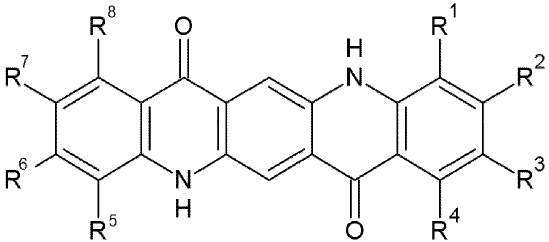
où les substituants R1 à R8 sont des atomes d'hydrogène, de fluor, de chlore ou de brome ou des radicaux alkyles en C1-C2 (-CH3 ou -CH2-CH3), et
où les dérivés disubstitués sur R1 et R5, R2 et R6, R3 et R7, et R4 et R8 sont présents de préférence sous forme d'isomères cis ou trans (isomères Z et E) en modification bêta ou gamma et sous forme de leurs tautomères.

14. Utilisation selon l'une quelconque des revendications 9 à 13, dans laquelle le composé quinacridone comprend des dérivés méthylés, des dérivés halogénés, des isomères cis-trans, des tautomères, ainsi que des variantes des quinacridones et de leurs dérivés, y compris leurs isomères et tautomères, dans les structures cristallines alpha I, alpha II, bêta, et gamma.
